# EUROPEAN PATENT SPECIFICATION

(11) **EP 4 147 637 B1**
(45) Date of publication and mention of the grant of the patent: **06.08.2025**
(21) Application number: 20934680.8
(22) Date of filing: 29.12.2020
(51) Int. Cl.: A61B 5/11, A61B 5/117, A61B 5/00

(54) **GAIT ANALYSIS SYSTEM AND METHOD**
GANGANALYSESYSTEM UND -VERFAHREN
SYSTÈME ET PROCÉDÉ D'ANALYSE DE LA DÉMARCHE

(30) Priority: 07.05.2020 KR 20200054386
(43) Date of publication of application: 15.03.2023
(73) Proprietor: Gwangju Institute of Science and Technology, Gwangju 61005 (KR)
(72) Inventor: KIM, Mun Sang, Gwangju 61005 (KR); LEE, Deok Won, Daejeon 35262 (KR); LEE, Sang Hyub, Goheung-gun Jeollanam-do 59511 (KR); JUN, Kook Sung, Incheon 21368 (KR)
(74) Representative: Botti & Ferrari S.p.A.
(86) International application number: PCT/KR2020/019341
(87) International publication number: WO 2021/225249

(56) References cited:
- WO-A1-2018/066421
- JP-A- 2010 017 447
- JP-A- 2016 140 591
- KR-A- 20100 004 967
- KR-B1- 101 373 628
- US-A1- 2015 325 004
- US-A1- 2017 238 846
- BEHNAM MALMIR: "Exploratory studies of human gait changes using depth cameras and considering measurement errors", ARXIV.ORG, CORNELL UNIVERSITY LIBRARY, 201 OLIN LIBRARY CORNELL UNIVERSITY ITHACA, NY 14853, 21 March 2019 (2019-03-21), XP081156387
- LEE DEOK-WON ET AL: "Abnormal Gait Recognition Using 3D Joint information of Multiple Kinects System and RNN-LSTM", 2019 41ST ANNUAL INTERNATIONAL CONFERENCE OF THE IEEE ENGINEERING IN MEDICINE AND BIOLOGY SOCIETY (EMBC), IEEE, 23 July 2019 (2019-07-23), pages 542 - 545, XP033625512, DOI: 10.1109/EMBC.2019.8857607

## Description

### BACKGROUND OF THE INVENTION

### Field of the Invention

The present disclosure relates to a system for analyzing walking behavior, the system being able to help experts propose medical opinions by determining problems with walking behavior.

### Description of the Related Art

People make efforts to optimally control their postures that are suitable for the environments inside and outside the body and specific subjects in everyday life, and such posture balance control is necessary and has an important meaning when we independently live.

In particular, movement of the center of gravity of posture balance control abilities can be shown not only in an erect posture, but various activities in everyday line, and the load that is applied in situations such as when a person lifts an object or walks with a bag on his/her shoulder has considerable influence on posture maintenance and balance maintenance.

When excessive load is applied while a person carries an object or when a person walks and carries an object in a wrong way, movement of the center of gravity is influenced by variation of the walking pattern. In normal walking, the center of gravity is regularly and smoothly changed up, down, left, right, and in the walking direction and the legs and arms are harmoniously moved. However, when a person walks with a load in his/her arms or on his/her shoulders, the center of gravity is irregularly moved.

Further, a coping mechanism for maintaining balance by bringing the center line of gravity, of which the position has been changed due to the way of carrying a load, to the center of base of support, for maintaining balance by bringing the center line further forward to move forward, and for reducing energy consumption for walking is activated in a human body.

Gait analysis is used in decision making in clinical tests, evaluation after treatment, evaluation of intention and helping tools, and various biomechanical studies for musculoskeletal and neurologic diseases, does an inclusive evaluation by measuring various features of walking, and includes kinematic analysis, dynamic electromyography, energy expenditure measurement, etc.

In general, walking pattern extraction devices are widely used for rehabilitation of recovering the walking sense of patients with a walking disorder due to injuries to the central nervous system, for walking posture correction training of users who need walking posture correction, or the like.

Meanwhile, according to walking analysis methods of the related art, it is required to attach attachable sensors (for example, inertia sensors such as a gyro sensor and an acceleration sensor) to the body, obtain information from the attachable sensors, and attach such attachable sensors for every measurement, so it is limited to analyze walking in everyday life.

Further, a walking analysis method using a single depth camera has limitation that a range in which walking data can be obtained is small and the accuracy of kinematic information of hidden body parts is deteriorated due to an occlusion phenomenon in which a body part is hidden by another body part.

Accordingly, the present disclosure intends to provide a system for analyzing walking behavior that can solve the problem that the accuracy of kinematic information of body parts is deteriorated due to the occlusion phenomenon.

US 2017/238846 A1 discloses a method for gait analysis of a subject performed periodically over time to detect changes in one or more gait characteristics.

### SUMMARY OF THE INVENTION

An objective of the present disclosure is to provide a system for analyzing walking behavior that can solve the problems of the related art.

In order to achieve the objectives, a system for analyzing walking behavior according to an embodiment of the present disclosure includes: an identifier configured to identify a walker; a walking information obtainer configured to image a walking motion of the walker in at least one or more directions of a left, a right, and a front on the basis of an identification signal from the identifier, and to extract and provide kinematic information from the taken images; a walking information collector configured to combine and collect kinematic information that is 3D and/or 2D skeleton information of the taken walking images; a walking parameter extractor/classifier configured to extract at least one or more walking parameter on the basis of the combined kinematic information that is 3D and/or 2D skeleton information, and then classify the kind of the walking; and a walking result informer configured to determine whether the at least one or more walking parameters come out of a recommendation reference value, or are included in a walking parameter range of abnormal walking, or are classified as a pathological gait, and then provide the result to the walker or an expert, wherein the walking information obtainer recognizes markers in which three axis points are recorded and that are arranged with regular intervals on a walking path when a walking motion is imaged by a plurality of cameras, and calculates a 3D and/or 2D transformation matrix of each kinematic information that is 3D and/or 2D skeleton information taken on the basis of the three axis points, and transforms axes of coordinates.

By using the system for analyzing walking behavior according to an embodiment of the present disclosure, it is possible to extract and classify walking parameters such as the step, the speed, the waist curvature, the average knee bending angle, etc. of a walker and to provide the classified walking motion of the walker such that the classified walking motion is used as a datum helping an expert propose a medical opinion.

### BRIEF DESCRIPTION OF THE DRAWINGS

The above and other objectives, features and other advantages of the present invention will be more clearly understood from the following detailed description when taken in conjunction with the accompanying drawings, in which:
FIG. 1 is a block diagram showing a system for analyzing walking behavior according to an embodiment of the present disclosure;

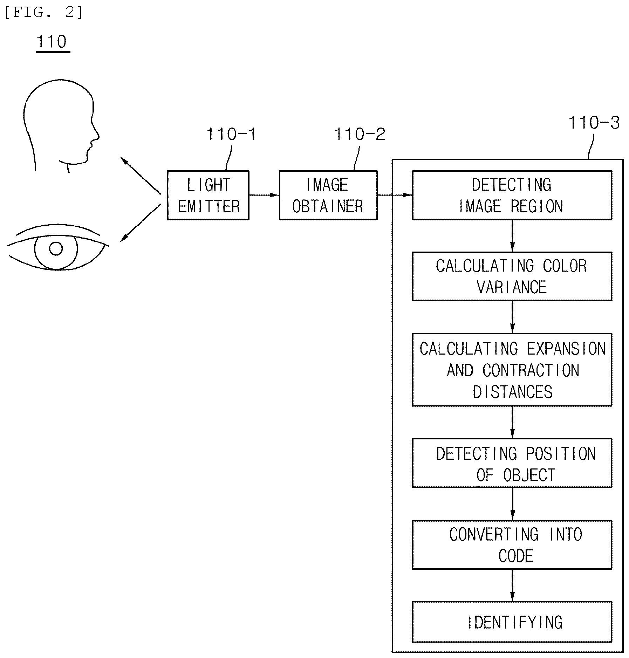

FIG. 2 is an exemplary view showing the configuration of an identifier shown in FIG. 1;

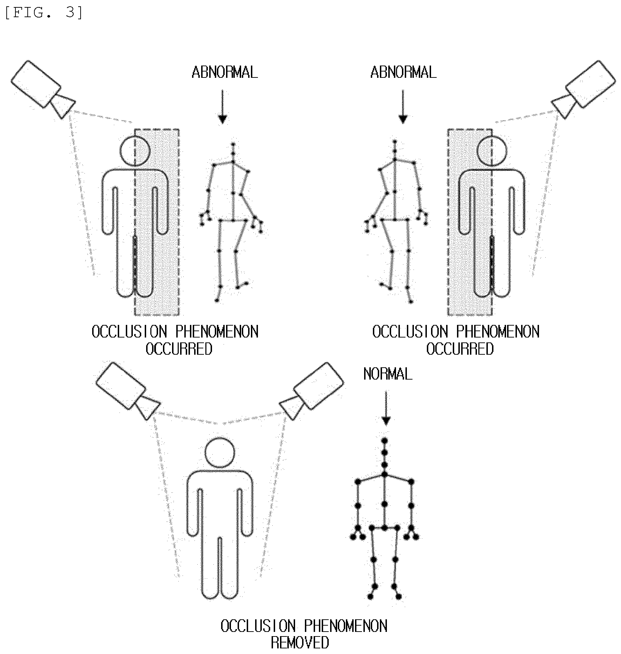

FIG. 3 is an exemplary view showing an example of kinematic information extracted from depth cameras arranged in parallel;

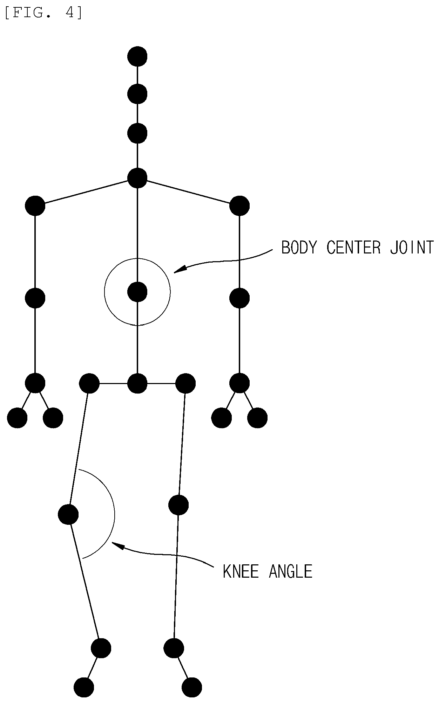

FIG. 4 is a view showing an example of kinematic information;

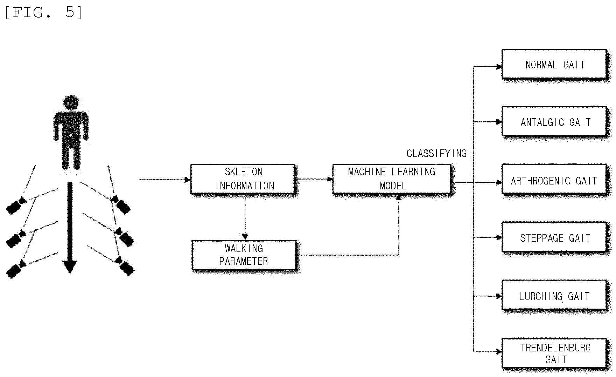

FIG. 5 is an exemplary view showing a process of classifying walking types according to continuous skeleton information using a mechanical learning model;

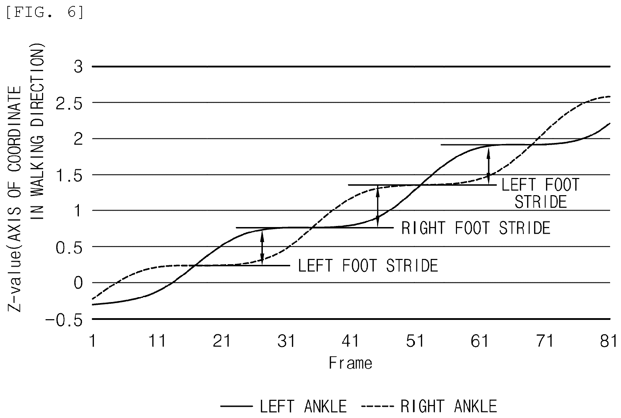

FIG. 6 is a table showing variations of a walking parameter (a step) that is one of kinematic information in a walking behavior image;
FIG. 7 is a table comparing variations of skeleton information (ankles) obtained by a single depth camera and a pair of depth cameras;
FIG. 8 is a flowchart showing a method of analyzing walking behavior by means of a system according to an embodiment of the present disclosure; and

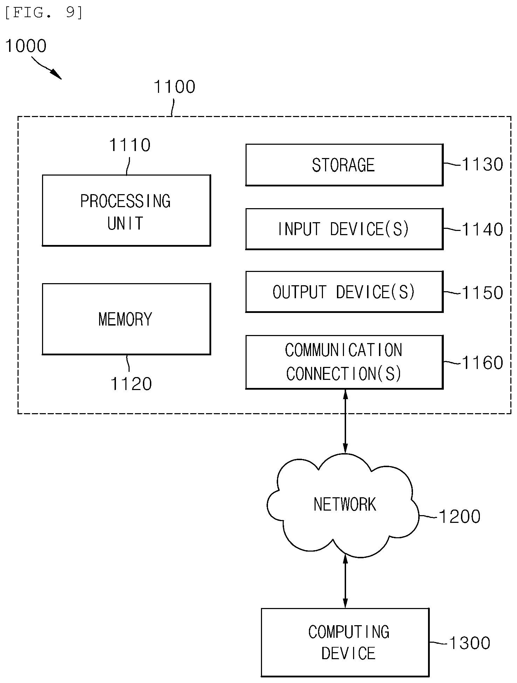

FIG. 9 is a view showing an exemplary computing environment in which one or more embodiments described herein can be implemented.

### DETAILED DESCRIPTION OF THE INVENTION

In order to achieve the objectives, a system for analyzing walking behavior according to an embodiment of the present disclosure includes: an identifier configured to identify a walker; a walking information obtainer configured to image a walking motion of the walker in at least one or more directions of a left, a right, and a front on the basis of an identification signal from the identifier, and to extract and provide kinematic information from the taken images; a walking information collector configured to combine and collect kinematic information (3D and/or 2D skeleton information) of the taken walking images; a walking parameter extractor/classifier configured to extract at least one or more walking parameter on the basis of the combined kinematic information (3D and/or 2D skeleton information), and then classify the kind of the walking; and a walking result informer configured to determine whether the at least one or more walking parameters come out of a recommendation reference value, or are included in a walking parameter range of abnormal walking, or are classified as a pathological gait, and then provide the result to the walker or an expert.

In order to achieve the objectives, a system for analyzing walking behavior according to an embodiment of the present disclosure can be used for performing: an identification step of identifying a walker by means of an identifier; a kinematic information extraction step of imaging a walking motion of the walker in at least one or more directions of a left, a right, and a front on the basis of an identification signal from the identifier, and then extracting and providing kinematic information from the taken images by means of a walking information collector; a combination step of combining kinematic information (3D and/or 2D skeleton information) of the walking images taken by a walking information collector; a walking parameter extraction and classification step of a extracting at least one or more walking parameter on the basis of the combined kinematic information (3D and/or 2D skeleton information), and then of classifying a walking type by means of a walking parameter extractor/classifier; and a walking problem determination step of determining a walking problem of a walker on the basis of the at least one or more walking parameters or the result of classifying walking by means of a walking result informer.

In the following description, the structural or functional description specified to exemplary embodiments according to the concept of the present disclosure is intended to describe the exemplary embodiments, so it should be understood that the present disclosure may be variously embodied, without being limited to the exemplary embodiments.

Embodiments described herein may be changed in various ways and various shapes, so specific embodiments are shown in the drawings and will be described in detail in this specification. However, it should be understood that the exemplary embodiments according to the concept of the present disclosure are not limited to the embodiments which will be described hereinbelow with reference to the accompanying drawings.

It will be understood that, although the terms first and/or second, etc. may be used herein to describe various elements, these elements should not be limited by these terms. These terms are only used to distinguish one element, from another element. For instance, a first element discussed below could be termed a second element without departing from the right range of the present disclosure. Similarly, the second element could also be termed the first element.

It is to be understood that when one element is referred to as being "connected to" or "coupled to" another element, it may be connected directly to or coupled directly to another element or be connected to or coupled to another element, having the other element intervening therebetween. On the other hand, it should to be understood that when one element is referred to as being "connected directly to" or "coupled directly to" another element, it may be connected to or coupled to another element without the other element intervening therebetween. Further, the terms used herein to describe a relationship between elements, that is, "between", "directly between", "adjacent" or "directly adjacent" should be interpreted in the same manner as those described above.

Terms used in the present disclosure are used only in order to describe specific exemplary embodiments rather than limiting the present disclosure. Singular forms are intended to include plural forms unless the context clearly indicates otherwise. It will be further understood that the terms "comprises" or "have" used in this specification, specify the presence of stated features, steps, operations, components, parts, or a combination thereof, but do not preclude the presence or addition of one or more other features, numerals, steps, operations, components, parts, or a combination thereof.

Unless defined otherwise, it is to be understood that all the terms used in the specification including technical and scientific terms has the same meaning as those that are understood by those who skilled in the art. It will be further understood that terms defined in dictionaries that are commonly used should be interpreted as having meanings that are consistent with their meanings in the context of the relevant art and will not be interpreted in an idealized or overly formal sense unless expressly so defined herein.

Hereafter, a system for analyzing walking behavior according to an embodiment of the present disclosure is described in more detail with reference the accompanying drawings.

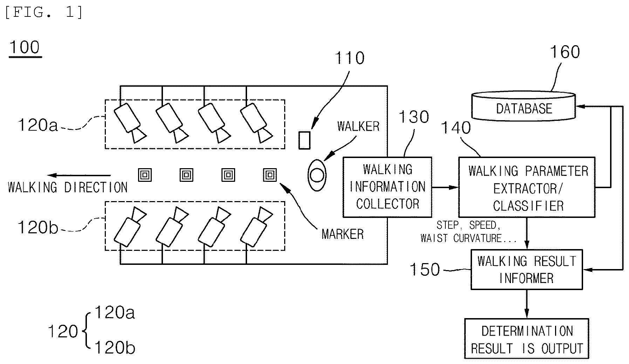

FIG. 1 is a block diagram showing a system for analyzing walking behavior according to an embodiment of the present disclosure.

As shown in FIG. 1, a system 100 for analyzing walking behavior according to an embodiment of the present disclosure includes an identifier 110, a walking information obtainer 120, a walking information collector 130, a walking parameter extractor/classifier 140, a walking result informer 150, and a database 160.

The identifier 110 may be a component that identifies a walker. The identifier 110 can identify a walker using the facial profile and eyeball information of the walker or can identify a walker using the facial profile, eyeball information, and voice information (voice) of the walker.

For example, when the identifier 110 uses eyeball information, the identifier 110 may be composed of a light emitter 110-1, an image obtainer 110-2, and an identity determiner 110-3 for detecting dilatation information of irises and pupils that respond to the brightness of variable light that is emitted for a predetermined time.

The light emitter 110-1 emits variable light to an eyeball for a predetermined time. The variable light may be emitted in shape of a specific symbol, letter, or number and the sizes of the specific symbol, letter, and number may be changed.

The image obtainer 110-2 detects iris and pupil regions in an eyeball image collected for a predetermined time.

The identity determiner 110-3 can detect an iris region and a pupil region from a plurality of eyeball image frames detected for a predetermined time, calculate and process a color variance according to the variable light into a color coordinate vector, detect expansion and contraction distances of the iris region and the pupil region according to the variable light, detect position information of a specific symbol, letter, or number projected into the iris region and changing in size, and convert the color coordinate vector, the expansion and contraction distances, and the position information into a 2D code.

The identity determiner 110-3 can determine the identity of a walker by determining whether the converted 2D code is the same as a pre-stored 2D code of a walker.

As another example, the identifier 110 may be a reader that can identify a walker by recognizing an ID card in which walker information is recorded in a QR code or an NFC tag.

Next, the walking information obtainer 110 may be composed of one or a plurality of 2D or depth (3D) cameras. When a plurality of cameras is used, 2D cameras or depth cameras (3D cameras) may be arranged in parallel in lines to be able to take pictures of the left side and the right side of a walker, respectively. Accordingly, two dept (3D) cameras or 2D cameras may be disposed on the same axis to face each other. However, the present disclosure is not limited to the arrangement of two or more cameras on the same axis and cameras may be randomly disposed in a space for measurement. The depth camera may be Kinect, Realsense, Astra, and the like, and an inertia sensor or motion capture equipment that can obtain 3D space information may replace the depth camera.

The sensor or equipment obtains kinematic information (2D or 3D skeleton and joint information) of a walker who is walking.

The kinematic information that is provided by the sensor or equipment can provide skeletal coordinates and skeletal reliability.

Each of the depth cameras can recognize information of the depth to a specific point from at least one or more markers or IR markers attached in a walking path of a walker.

Each of the depth cameras recognize an axis point (coordinate axis) recorded on the marker positioned at a predetermined imaging point.

The axis point is used in a calibration (combination) process of kinetic information by an image combiner to be described below.

The walking information obtainer 110 may include a coordinate sensor that traces positions (coordinates) of markers in a set imaging point when the imaging points of a plurality of depth cameras or 2D cameras due to external factors.

The walking information collector 130 collects kinematic information (3D and/or 2D skeleton information) according to walking of a target obtained by a plurality of depth cameras, 2D cameras, inertia sensors, or motion capture equipment, classifies in detail kinematic information (3D and/or 2D skeleton information) of cameras positioned in the same axial direction, and calibrates (combines) kinematic information (3D and/or 2D skeleton information) extracted from images taken at positions of the same axis.

The calibration (combination and modification) process may be process of transforming the axis of coordinates of each frame on the basis of three or more axis points obtained through markers, calculating 3D and/or 2D matrixes of the transformed axes of coordinates, and then projecting kinematic information (3D and/or 2D skeleton information and joint information) extracted from a plurality of images to one axis of coordinates.

For example, assuming that a total of four depth cameras are disposed at the left and right sides of a preset walking path and a walker has walked along the walk path for 0 ~ 8 seconds, when depth camera Nos. 1 and 2 provide kinematic information of the walker for 0 ~ 3 seconds and depth camera Nos. 3 and 4 provide kinematic information of the walker for 2.5 ~ 5.5 seconds, left/right kinematic information of each of (1, 2) and (3, 4) may be combined and front/rear kinematic information of (1, 3) and (2, 4) may be combined.

As another example, assuming that a total of four dept cameras are arranged with regular intervals at a side of a preset walking path and a walker has walked along the walk path for 0 ~ 8 seconds, kinematic information of the walker taken by the four cameras at each time section may be combined front and rear.

The walking path may be an actual space having a predetermined length or may be a machine such as a treadmill enabling continuous walking.

Next, the walking parameter extractor/classifier 140 may be a component that extracts walking parameters from the calibrated kinematic information (3D and/or 2D skeleton information and then classifies the types of walking using the extracted walking parameters or continuous skeleton information or uses the extracted walking parameters or continuous skeleton information as input data for machine learning.

The walking parameter extractor/classifier 140 can classify the extracted walking parameters or continuous skeleton information as any one of types of walking, such as a normal gait, an antlgic gait, an arthrogenic gait, a steppage gait due to weakening of the shin muscles, a lurching gait due to weakening of the gluteus maximus muscle, a trendelenburg gait due to weakening of the gluteus medius muscle, and a pathological gait, using a machine learning model, for example, an algorithm such as CNN, RNN, K-NN, K-MEANS, and RANDOM FOREST. The types of walking described herein are only examples.

For example, when walking is classified as an arthrogenic gait, there is a possibility of a problem with the ankles, the knees, or the pelves, so the classified walking type can greatly help experts propose medical opinions. When walking is classified into a pathological gait, it may be used as information for persuading the walker to visit a hospital.

The walking parameters or continuous skeleton information itself extracted from the walking parameter extractor/classifier may be used as an index for determining a problem with walking. The walking parameters may be a step, a speed, the curvature of a waist, left and right knee angles, an ankle angle, a pelvis twist angle, etc.

The walking speed is calculated as a movement distance/movement time of a body center joint, the step is calculated a z-axial distance between stop sections of both ankles (Z-axial direction: walking direction), the knee bending angle is calculated as the average value of the angles made of thigh joint-knee joint-ankle joint, and the curvature of a waist is calculated as the angle made by a vector connecting the head-body centers during walking in an absolute coordinate system.

The walking result informer 150 determines whether the at least one or more walking parameters come out of a recommendation reference value, or are included in a walking parameter range of abnormal walking, or are classified as the pathological gait, and then provides the result to the walker or an expert.

The walking result informer 150 determines whether there is a problem with walking of a walker using a recommendation value of each walking parameter.

The walking result informer 150 may use, as references for determination, ① how much a walking parameter comes out of a recommendation value, ② how much a walking parameter has changed from a normal state, ③ whether walking of a walker is classified as the pathological gait (whether a classified result is the pathological gait when entire joint information or walking parameters are input to a machine learning model or when walking is the pathological gait when walking parameter themselves are analyzed), etc.

For example, walking may be classified into two classes of a normal gait and a pathological gait, or may be classified into a normal gait, a pathological gait 1, a pathological gait 2, ..., and pathological gait n. That is, the range of the normal gait may also be classified into n classes that a user wants, so the reference may depend in situations.

The walking result informer 150 can determine various walking problems in accordance with the number of times of walking

### - Example that can determined as one time of walking -

1) A problem with walking speed; a problem is sensed when the difference from the average walking speed for each age group, 2) a problem with a step size: it is sensed when the difference from a recommendation step (height - 1m) is 20% or more, 3) sensing of left-right step unbalance: it is sensed when the difference between a right step and a left step is 20cm or more, 4) front-rear waist curvature: it is sensed when the front/rear curvature of a waist is 10 degrees or more, 5) left-right waist curvature: it is sensed when the left/right curvature of a waist is 10 degrees or more, and 6) walking classification: a problem is determined when a classified walking type is a pathological gait, etc.

### - Example that can be determined as a several times of walking -

1) A problem with walking speed: when a walking speed is different from a normal walking speed by 10% or more or gradually decreases, 2) a problem with a step: when a step is different from a normal step by 10% or more or gradually decreases, 3) sensing of left-right step unbalance: when the difference between left and right steps is 10% or more or gradually increases, 4) front-rear waist curvature: when the front/rear curvature of a waist is different from a normal state by 10% or more or gradually increases, 5) left-right waist curvature: when the left/right curvature of a waist is different from a normal state by 10% or more or gradually increases, and 6) knee bending angle: when the knee bending angle is different from the normal state by 10% or more or gradually decreases, etc.

Meanwhile, the walking information collector 130, the walking parameter extractor/classifier 140, and the walking result informer 150 disclosed the present disclosure may be operated in cooperation with the database 160.

The database 160 can store the information collected, extracted, and determined by the components described above, and can upload existing stored information.

The database 160 may include fields or elements such that the database 160 can be implemented to be fitted to the purpose of the present disclosure using a relational database management system (RDBMS) such as Oracle, MYSQL, MSSQL, Infomix, Sybase, and DB2, or an object-oriented database management system (OODBMS) such as Gemston, Orion, and O2, and an XML native database such as Excelon, Tamino, and Sekainu.

Each field or element may be formed as a field or element of a superordinate concept or a subordinate concept.

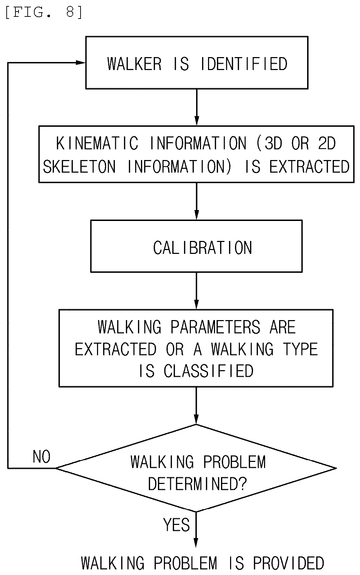

FIG. 8 is a flowchart showing a method of analyzing walking behavior by means of a system according to an embodiment of the present disclosure.

Such a method of analyzing walking behavior (S700), first, when a walker is identified (S710), extracts kinematic information of the walker in walking images by means of the walking information obtainer 120 (S720).

The walking information collector 130 collects kinematic information (3D and/or 2D skeleton information) according to walking of a target obtained by a single or a plurality of depth cameras, 2D cameras, inertia sensors, or motion capture equipment, classifies in detail kinematic information (3D and/or 2D skeleton information) of cameras positioned in the same axial direction, and calibrates (combines) kinematic information (3D and/or 2D skeleton information) extracted from images taken at positions of the same axis (S730).

The calibration (combination and modification) process may be process of transforming the axis of coordinates of each frame on the basis of three axis points obtained through markers, calculating 3D and/or 2D matrixes of the transformed axes of coordinates, and then projecting kinematic information (3D and/or 2D skeleton information and joint information) extracted from a plurality of images to one axis of coordinates.

For example, assuming that a total of four depth cameras are disposed at the left and right sides of a preset walking path and a walker has walked along the walk path for 0 ~ 8 seconds, when depth camera Nos. 1 and 2 provide kinematic information of the walker for 0 ~ 3 seconds and depth camera Nos. 3 and 4 provide kinematic information of the walker for 2.5 ~ 5.5 seconds, left/right kinematic information of each of (1, 2) and (3, 4) may be combined and front/rear kinematic information of (1, 3) and (2, 4) may be combined.

As another example, assuming that a total of four dept cameras are arranged with regular intervals at a side of a preset walking path and a walker has walked along the walk path for 0 ~ 8 seconds, kinematic information of the walker taken by the four cameras at each time section may be combined front and rear.

Meanwhile, the process S730 may include a process of calculating reliability of the coordinates of a skeleton and a process of combining only items of kinematic information of which the reliability of coordinates is a reference value or more.

Thereafter, when the process S730 is finished, the walking parameter extractor/classifier 140 extracts and classifies walking parameters on the basis of the calibrated kinematic information (3D and/or 2D skeleton information) (S740).

For example, the process S740 may include a process of classifying the extracted walking parameters or continuous skeleton information as any one of types of walking, such as a normal gait, an antalgic gait, an arthrogenic gait, a steppage gait due to weakening of the shin muscles, a lurching gait due to weakening of the gluteus maximus muscle, a trendelenburg gait due to weakening of the gluteus medius muscle, and a pathological gait, using a machine learning model, for example, an algorithm such as CNN, RNN, K-NN, K-MEANS, and RANDOM FOREST. The types of walking described herein are only examples.

For example, when walking is classified as an arthrogenic gait, there is a possibility of a problem with the ankles, the knees, or the pelves, so the classified walking type can greatly help experts propose medical opinions. When walking is classified into a pathological gait, it may be used as information for persuading the walker to visit a hospital.

The extracted walking parameters or continuous skeleton information itself may be used as an index for determining a problem with walking or may be input to a machine learning model. The walking parameters may include at least one or more of a walking speed, the height of a walker, movement of hands during walking, the length of a step, a front-rear waist curvature, a left-right waist curvature, a knee bending angle, angles of joints, and the times of a stance phase and a swing phase, but are not limited thereto.

The walking speed is calculated as a movement distance/movement time of a body center joint, the step is calculated a z-axial distance between stop sections of both ankles (Z-axial direction: walking direction), the knee bending angle is calculated as the average value of the angles made of thigh joint-knee joint-ankle joint, and the curvature of a waist is calculated as the angle made by a vector connecting the head-body centers during walking in an absolute coordinate system.

When the process S740 is finished, the walking result informer 150 determines whether there is a problem with walking of the walker on the basis of the walking parameters (S750).

The process S750, which is a process of determining whether there is a problem with walking of a walker using a recommendation value for each walking parameter, is a process of determining various walking problems in accordance with the number of times of walking.

The process S750 may use, as references for determination, ① how much a walking parameter comes out of a recommendation value, ② how much a walking parameter has changed from a normal state, ③ whether walking of a walker is classified as the pathological gait (whether a classified result is the pathological gait when entire joint information or walking parameters are input to a machine learning model or when walking is the pathological gait when walking parameter themselves are analyzed), etc.

For example, walking may be classified into two classes of a normal gait and a pathological gait, or may be classified into a normal gait, a pathological gait 1, a pathological gait 2, ..., and pathological gait n. That is, the range of the normal gait may also be classified into n classes that a user wants, so the reference may depend in situations.

### - Example that can determined as one time of walking -

1) A problem with walking speed; a problem is sensed when the difference from the average walking speed for each age group, 2) a problem with a step size: it is sensed when the difference from a recommendation step (height - 1m) is 20% or more, 3) sensing of left-right step unbalance: it is sensed when the difference between a right step and a left step is 20cm or more, 4) front-rear waist curvature: it is sensed when the front/rear curvature of a waist is 10 degrees or more, 5) left-right waist curvature: it is sensed when the left/right curvature of a waist is 10 degrees or more, and 6) walking classification: a problem is determined when a classified walking type is a pathological gait, etc.

### - Example that can be determined as a several times of walking -

1) A problem with walking speed: when a walking speed is different from a normal walking speed by 10% or more or gradually decreases, 2) a problem with a step: when a step is different from a normal step by 10% or more or gradually decreases, 3) sensing of left-right step unbalance: when the difference between left and right steps is 10% or more or gradually increases, 4) front-rear waist curvature: when the front/rear curvature of a waist is different from a normal state by 10% or more or gradually increases, 5) left-right waist curvature: when the left/right curvature of a waist is different from a normal state by 10% or more or gradually increases, and 6) knee bending angle: when the knee bending angle is different from the normal state by 10% or more or gradually decreases, etc.

By using the system for analyzing walking behavior according to an embodiment of the present disclosure, there is an advantage that it is possible to more accurately measure the step, the speed, the waist curvature, the average knee bending angle, etc. of a walker and it is possible to determine whether there is a problem with walking of the walker on the basis of these items of information.

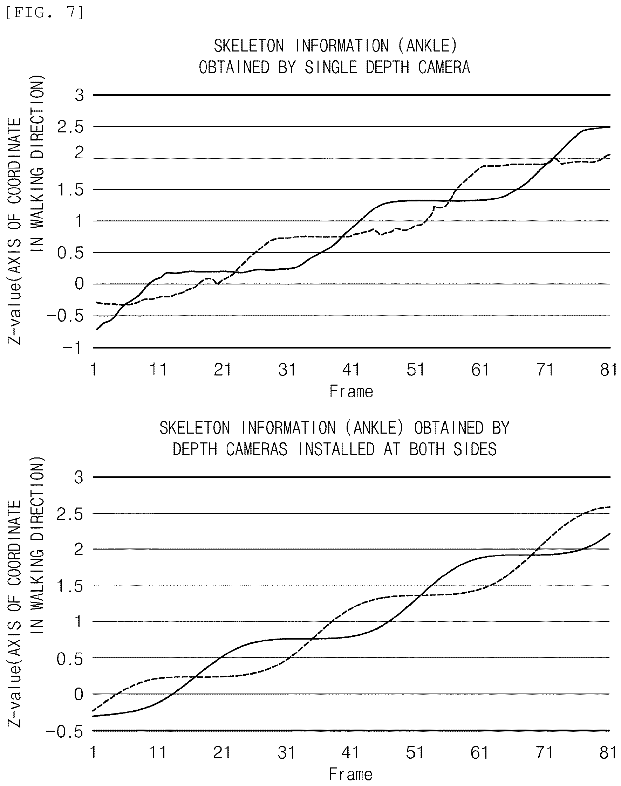

FIG. 7 is a view showing an exemplary computing environment in which one embodiment disclosed herein can be implemented, in which a system 1000 including a computing device 1100 configured to implement the one or more embodiments described above is exemplified. For example, the computing device 1100 includes a personal computer, a server computer, a hand-held or laptop device, a mobile device (a mobile phone, a PDA, a media player, etc.), a multiprocessor system, a consumer electronic device, a mini computer, a main frame computer, and a distribution computing environment including these systems or devices, but is not limited thereto.

The computing device 1100 may include at least one processing unit 1110 and memory 1120. The processing unit 1110, for example, may include a central processing unit, a graphic processing unit, a microprocessor, an Application Specific Integrated Circuit (ASIC), a Field Programmable Gate Arrays (FPGA), etc., and may have a plurality of cores. The memory 1120 may be a volatile memory (e.g., a RAM, etc.), a nonvolatile memory (e.g., a ROM, a flash memory, etc.), or a combination thereof. The computing device 1100 may include an additional storage 1130. The storage 1130 includes a magnetic storage, an optical storage, etc., but is not limited thereto. In the storage 1130, computer-readable instructions for implementing one or more of embodiments disclosed herein may be stored, and other computer-readable instructions for implementing an operating system, application programs, etc. may also be included. The computer-readable instructions stored in the storage 1130 can be loaded to the memory 1120 to be executed by a processing unit 1110. The computer device 1110 may further includes an input device(s) 1140 and an output device(s) 1150.

The input device(s) 1140, for example, may include a keyboard, a mouse, a pen, a voice input device, a touch input device, an infrared camera, a video input device, or any other input devices. The output device(s) 1150, for example, may include one or more displays, speakers, printers, or any other output devices. The computing device 1100 may include an input device or an output device of another computer device as the input device(s) 1140 or the output device (s) 1150. The computing device 1100 may further include a communication connection(s) 1160 enabling the computing device 1100 to communicate with another device (e.g., a computing device 1300).

The communication connection(s) 1160 may include a modem, a network interface card (NIC), an integrated network interface, a wireless frequency transmitter/receiver, an infrared port, a USB connection, or other interfaces for connecting the computing device 1100 to another computing device. The communication connection(s) 1160 may further include a wired communication or a wireless communication. The components of the computing device 1100 may be connected by various interconnections such as a bus (e.g., a periphery component interconnection (PCI), a USB, firmware (IEEE 1394, an optical bus structure, etc.), and may be interconnected by a network 1200. The terms 'component', 'system', etc. used herein generally means hardware, a combination of hardware and software, software, or a computer-related entity that is software that is being executed.

For example, a component may be a process that is being executed by a processor, a processor, an object, an executable matter, an execution thread, a program, and/or a computer, but is not limited thereto. For example, an application that is being executed by a controller and the controller both may be components. One or more components may exist in a process and/or an execution thread, and components may be localized in one computer or may be distributed between two or more computers.

The present disclosure is not limited to the embodiments described above and the accompanying drawings.

## Claims

1. A system (100) for analyzing walking behavior, comprising
an identifier (110) configured to identify a walker;
a walking information obtainer (120) configured to image a walking motion of the walker in at least one or more directions of a left, a right, and a front on the basis of an identification signal from the identifier (110), and to extract and provide kinematic information from the taken images;
a walking information collector (130) configured to combine and collect kinematic information that is 3D and/or 2D skeleton information of the taken walking images;
a walking parameter extractor/classifier (140) configured to extract at least one or more walking parameter on the basis of the combined kinematic information that is 3D and/or 2D skeleton information, and then classify the kind of the walking using the extracted walking parameter or continuous skeleton information or use the extracted walking parameter or continuous skeleton information as input data for machine learning; and
a walking result informer (150) configured to determine whether the at least one or more walking parameters come out of a recommendation reference value, or are included in a walking parameter range of abnormal walking, or are classified as a pathological gait, and then provide the result to the walker or an expert,
**characterized in that** the walking information obtainer (120)
recognizes markers in which three axis points are recorded and that are arranged with regular intervals on a walking path when a walking motion is imaged by a plurality of cameras, and
calculates a 3D and/or 2D transformation matrix of each kinematic information that is 3D and/or 2D skeleton information taken on the basis of the three axis points, and transforms axes of coordinates.

2. The system (100) of claim 1, wherein the walking information obtainer (120) images a walking motion of a walker using a device that can extract spatial imagelogical information of a human body such as one or a plurality of cameras, 2D cameras, inertia cameras, or motion capture equipment.

3. The system (100) of claim 1, wherein the walking information collector (130) performs combination on the basis of three or more axis points of the kinematic information that is 3D and/or 2D skeleton information when collecting information through a plurality of cameras.

4. The system (100) of claim 3, wherein the walking information collector (130) combines kinematic information having reliability over a reference value of left/right or front/rear kinematic information measured at the same time when imaging a walking motion through a plurality of cameras.

5. The system (100) of claim 4, wherein the walking parameters include at least one or more of a walking speed, the height of a walker, movement of hands during walking, the length of a step, a front-rear waist curvature, a left-right waist curvature, a knee bending angle, angles of joints, and the times of a stance phase and a swing phase.

6. The system (100) of claim 1, wherein the walking result informer (150) determines a walking problem on the basis of how much the at least one or more walking parameters are different from recommendation values.

7. The system (100) of claim 1, wherein the walking result informer (150) determines a walking problem on the basis of how much the walking parameters have changed from a normal state.

## Patentansprüche

1. System (100) zum Analysieren eines Gehverhaltens, umfassend
eine Identifiziereinrichtung (110), die dazu konfiguriert ist, eine gehende Person zu identifizieren;
eine Gehinformationserhalteeinrichtung (120), die dazu konfiguriert ist, eine Gehbewegung der gehenden Person in wenigstens einer oder mehreren Richtungen von einer linken, einer rechten und einer vorderen auf der Basis eines Identifikationssignals von der Identifiziereinrichtung (110) abzubilden, und aus den aufgenommenen Bildern kinematische Informationen zu extrahieren und bereitzustellen;
eine Gehinformationssammeleinrichtung (130), die dazu konfiguriert ist, kinematische Informationen, die 3D- und/oder 2D-Skelett-Informationen sind, der aufgenommenen Gehbilder zu kombinieren und zu sammeln;
eine Gehparameterextrahier-/klassifiziereinrichtung (140), die dazu konfiguriert ist, wenigstens einen oder mehrere Gehparameter auf der Basis der kombinerten kinematischen Informationen, die 3D- und/oder 2D-Skelett-Informationen sind, zu extrahieren und dann die Art des Gehens unter Verwendung des extrahierten Gehparameters oder kontinuierlicher Skelett-Informationen zu klassifizieren oder den extrahierten Gehparameter oder die kontinuierlichen Skelett-Informationen als Eingangsdaten für maschinelles Lernen zu verwenden; und
eine Gehergebnisinformationseinrichtung (150), die dazu konfiguriert ist, zu bestimmen, ob der wenigstens eine oder die mehreren Gehparameter aus einem Empfehlungsreferenzwert kommen, oder in einem Gehparameterbereich eines abnormalen Gehens enthalten sind, oder als ein pathologischer Gang klassifiziert sind, und dann das Ergebnis einer gehenden Person oder einem Experten bereitzustellen,
**dadurch gekennzeichnet, dass** die Gehinformationserhalteeinrichtung (120) Marker erkennt, an denen drei Achspunkte aufgezeichnet werden und die mit regulären Intervallen auf einem Gehpfad angeordnet sind, wenn eine Gehbewegung durch eine Vielzahl von Kameras abgebildet wird, und
eine 3D- und/oder 2D-Transformationsmatrix von jeder kinematischen Information berechnet, die eine 3D- und/oder 2D-Skelett-Information ist, die auf der Basis der drei Achspunkte aufgenommen wurde, und Koordinatenachsen transformiert.

2. System (100) nach Anspruch 1, wobei die Gehinformationserhalteeinrichtung (120) eine Gehbewegung einer gehenden Person unter Verwendung einer Vorrichtung abbildet, die räumliche bildlogische Informationen eines menschlichen Körpers extrahieren kann, wie etwa einer oder einer Vielzahl von Kameras, von 2D-Kameras, von Trägheitskameras, oder einer Bewegungserfassungsausrüstung.

3. System (100) nach Anspruch 1, wobei die Gehinformationssammeleinrichtung (130) eine Kombination auf der Basis von drei oder mehr Achspunkten der kinematischen Informationen, die 3D- und/oder 2D-Skelett-Informationen sind, beim Sammeln von Informationen durch eine Vielzahl von Kameras durchführt.

4. System (100) nach Anspruch 3, wobei die Gehinformationssammeleinrichtung (130) kinematische Informationen kombiniert, die eine Zuverlässigkeit gegenüber einem Referenzwert von Links-/Rechts- oder Vorne/Hinten-Kinematik-Informationen aufweisen, die zu derselben Zeit beim Abbilden einer Gehbewegung durch eine Vielzahl von Kameras gemessen wurden.

5. System (100) nach Anspruch 4, wobei die Gehparameter wenigstens einen oder mehrere einer Gehgeschwindigkeit, der Höhe einer gehenden Person, einer Bewegung von Händen während des Gehens, der Länge eines Schritts, einer Vorne-Hinten-Taillenkrümmung, einer Links-/Rechts-Taillenkrümmung, eines Kniebeugewinkels, von Winkeln von Gelenken, und den Zeiten der Standphase und einer Schwungphase umfassen.

6. System (100) nach Anspruch 1, wobei die Gehergebnisinformationseinrichtung (150) ein Gehproblem basierend darauf bestimmt, um wieviel der wenigstens eine oder die mehreren Gehparameter unterschiedlich von Empfehlungswerten sind.

7. System (100) nach Anspruch 1, wobei die Gehergebnisinformationseinrichtung (150) ein Gehproblem basierend darauf bestimmt, um wieviel sich die Gehparameter von einem Normalzustand geändert haben.

## Revendications

1. Système (100) pour analyser le comportement de marche, comprenant
un identifiant (110) configuré pour identifier un marcheur;
un récupérateur d'informations de marche (120) configuré pour imager un mouvement de marche du marcheur dans au moins une ou plusieurs directions de gauche, de droite et de devant sur la base d'un signal d'identification provenant de l'identifiant (110) et pour extraire et fournir des informations cinématiques à partir des images prises;
un collecteur d'informations de marche (130) configuré pour combiner et collecter des informations cinématiques qui sont des informations squelettiques 3D et/ou 2D des images de marche prises;
un extracteur/classificateur de paramètres de marche (140) configuré pour extraire au moins un ou plusieurs paramètres de marche sur la base des informations cinématiques combinées qui sont des informations squelettiques 3D et/ou 2D, et ensuite classer le type de marche en utilisant le paramètre de marche extrait ou les informations squelettiques continues ou utiliser le paramètre de marche extrait ou les informations squelettiques continues comme données d'entrée pour l'apprentissage automatique; et
un informateur de résultats de marche (150) configuré pour déterminer si l'au moins un ou plusieurs paramètres de marche proviennent d'une valeur de référence de recommandation, ou ils sont inclus dans une plage de paramètres de marche anormale, ou ils sont classés comme une démarche pathologique, et ensuite fournir le résultat au marcheur ou à un expert,
**caractérisé en ce que** le récupérateur d'informations de marche (120)
reconnaît des marqueurs dans lesquels trois points d'axe sont enregistrés et disposés à intervalles régulières sur un chemin de marche lorsqu'un mouvement de marche est imagé par une pluralité de caméras, et
calcule une matrice de transformation 3D et/ou 2D de chaque information cinématique qui est une information squelettique 3D et/ou 2D prise sur la base des trois points d'axe et transforme des axes de coordonnées.

2. Système (100) selon la revendication 1, dans lequel le récupérateur d'informations de marche (120) image un mouvement de marche d'un marcheur en utilisant un dispositif capable d'extraire des informations d'imagerie spatiale d'un corps humain, tels qu'une ou une pluralité de caméras, caméras 2D, caméras à inertie ou équipements de capture de mouvement.

3. Système (100) selon la revendication 1, dans lequel le collecteur d'informations de marche (130) effectue une combinaison sur la base de trois ou plusieurs points d'axe des informations cinématiques qui sont des informations squelettiques 3D et/ou 2D lors de la collecte d'informations par une pluralité de caméras.

4. Système (100) selon la revendication 3, dans lequel le collecteur d'informations de marche (130) combine des informations cinématiques ayant une fiabilité sur une valeur de référence d'informations cinématiques de gauche/droite ou avant/arrière mesurées en même temps lors de l'imagerie d'un mouvement de marche par une pluralité de caméras.

5. Système (100) selon la revendication 4, dans lequel les paramètres de marche comprennent au moins un/une ou plusieurs parmi la vitesse de marche, la hauteur d'un marcheur, le mouvement des mains pendant la marche, la longueur d'un pas, une courbure de la taille avant-arrière, une courbure de la taille gauche-droite, un angle de flexion du genou, les angles des articulations et les temps d'une phase d'appui et d'une phase d'oscillation.

6. Système (100) selon la revendication 1, dans lequel l'informateur de résultats de marche (150) détermine un problème de marche sur la base de la mesure dans laquelle au moins un ou plusieurs paramètres de marche diffèrent des valeurs recommandées.

7. Système (100) selon la revendication 1, dans lequel l'informateur de résultats de marche (150) détermine un problème de marche sur la base de la mesure dans laquelle les paramètres de marche ont changé par rapport à un état normal.
